# EUROPEAN PATENT APPLICATION

(11) **EP 4 056 162 A2**
(43) Date of publication of application: **14.09.2022**
(21) Application number: 20883746.8
(22) Date of filing: 07.11.2020
(51) Int. Cl.: A61H 31/00

(54) **CHEST COMPRESSION DEVICE**

(30) Priority: 07.11.2019 KR 20190142111
(71) Applicant: Cho, Young Beum, Dongdaemun-gu Seoul 02501 (KR)
(72) Inventor: CHO, Wook Hyun, Seoul 02501 (KR)
(74) Representative: Louis Pöhlau Lohrentz
(86) International application number: PCT/KR2020/015561
(87) International publication number: WO 2021/091332

(57) **Abstract**

Disclosed is a chest compression device.

## Description

### Technical field

The present invention relates to a chest compression device used for cardiopulmonary resuscitation.

### Background art

Cardiopulmonary resuscitation (CPR) is a first aid technique of regularly compressing the heart to circulate blood to the brain when the heart stops beating, thereby preventing brain cell damage. When the heart abruptly stops functioning, leading to a sudden cardiac arrest (SCA), a witness nearby should immediately give first aid by performing CPR. If CPR is not performed, a patient with SCA may be dead or brain dead.

CPR should be continuously repeated until the heart of the cardiac arrest patient beats again. It is not easy for ordinary people to continuously compress the heart of the cardiac arrest patient at a constant rate and at a constant pressure. Even paramedics or medical personnel who got professional training may have a physical difficulty in repeating CPR for a long time to compress the heart of the cardiac arrest patient.

Therefore, it is required to develop a mechanical device capable of compressing the heart of a cardiac arrest patient at a constant rate and at a constant pressure. Currently, Lucas^{™} portable chest compression devices are commercially available and used.

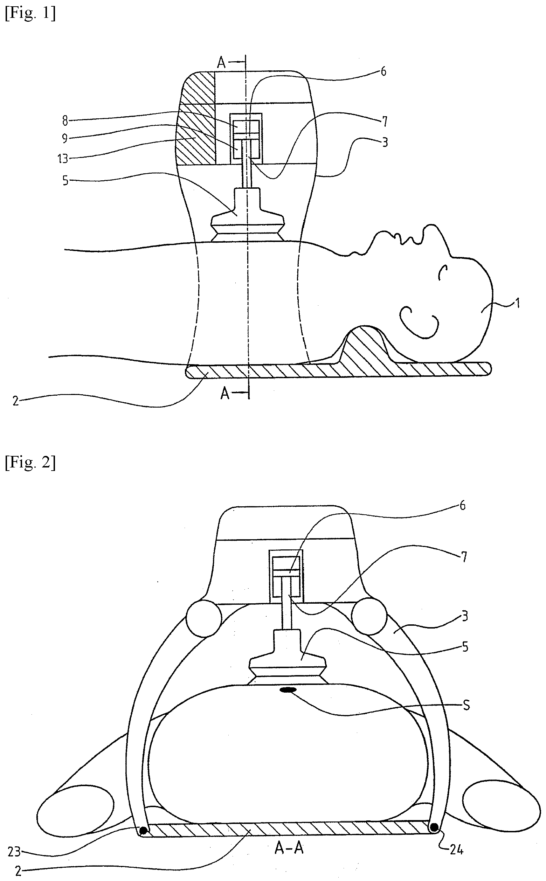

The conventional portable chest compression device as above is disclosed in Korean Patent Laid-open No. 2006-0009014 and is explained with reference to Figs. 1 and 2 attached herewith. Fig. 1 is a side view of a conventional chest compression device, and Fig. 2 is a cross-sectional view taken along the line A-A. The device includes a back 2 and a U-shaped support 3. A cardiac arrest patient 1 is straightly laid on his back on the back 2, and then the center of the back 2 is placed below the breastbone S. The back 2 is extended laterally to both sides of the patient. One extension part 23 of the back 2 is connected to one end of the support 3 by a hinge, and a snap lock for storing the other end (free end) of the support 3 is mounted on the other extension part 24 of the back, thereby rapidly fixing the support 3 on the back 2. Accordingly, the chest of the patient 1 is surrounded by the chest compression device. As illustrated in Fig. 2, the chest compression device has a shape similar to a stirrup. The central part of the U-shaped support 3 is positioned, maintaining a distance from the breastbone S.

The central part of the support 3 includes a compression unit. The compression unit includes a compression pad 5 connected to a piston 6 within the same chamber 8, 9 of a cylinder by a rod 7. The rate, distance, pressure, etc. of the reciprocating movement of the piston 6 and the compression pad 5 are controlled by a control unit 13 including a microprocessor. In general, the piston 6 is controlled to perform a vertical reciprocating movement at a constant rate within a range between 60 and 150 strokes per minute.

The device may be driven by a battery (not illustrated) provided therein, or may be driven by an external power source (not illustrated) by being connected to the external power source via a cable (not illustrated).

It is known that about 60-70% of cardiac arrest patients have cardiovascular problems which lead to cardiac arrest. Therefore, when the cardiac arrest patient visits a hospital, the doctor may need to monitor blood vessels of the heart using coronary angiography (pPCI) in order to examine cardiovascular problems.

However, while performing CPR using the conventional chest compression device or performing CPR by a person, the heart is hidden by the chest compression device or the person performing CPR, and thus cardiovascular images cannot be obtained using coronary angiography.

### Detailed description of invention

### Technical task

In order to solve the above-mentioned problems, it is an object of the present invention to provide a chest compression device capable of obtaining cardiovascular images using coronary angiography even during chest compression.

### Means for solving technical task

The chest compression device of the present invention for solving the above task includes a back for supporting the back of a patient; a compression part for compressing the chest of the patient; a U-shaped support for supporting the compression part; a pair of driving parts for allowing the support and the compression part connected thereto to perform a vertical reciprocating movement; and a control part for controlling the operation of the pair of driving parts.

According to the present invention, preferably, the support and the compression part are made of materials through which X-rays may penetrate (for example, plastic materials).

According to the present invention, preferably, one end of a first driving part among the pair of driving parts is connected to one end of the support, and the other end of the first driving part is connected to one end of the back, and one end of a second driving part among the pair of driving parts is connected to the other end of the support, and the other end of the second driving part is connected to the other end of the back.

According to one embodiment of the chest compression device of the present invention, each of the pair of driving parts may be provided with a rod performing a vertical linear movement, and each of the pair of driving parts may be supported by the back.

Here, the distal end of the rod of the first driving part among the pair of driving parts may be connected to one end of the support, the first driving part may be connected to and supported by one end of the back, the distal end of the rod of the second driving part among the pair of driving parts may be connected to the other end of the support, and the second driving part may be connected to and supported by the other end of the back.

According to the present invention, the control part may control the vertical reciprocating movements of both ends of the support by the pair of driving parts to be synchronized with each other.

### Effect of invention

In the chest compression device according to the present invention, the driving part allowing the compression part and the support connected thereto to perform a vertical reciprocating movement is not located in the center of the support, but is located at both bottom ends of the support. Therefore, cardiovascular images may be obtained using coronary angiography while CPR is performed with the chest compression device according to the present invention. Accordingly, it is possible to monitor the blood vessels of the heart using coronary angiography simultaneously with chest compression on the cardiac arrest patient using the chest compression device according to the present invention.

### Brief description of drawings

Figs. 1 and 2 are a perspective view and a cross-sectional view of a known chest compression device;
Fig. 3 is a perspective view of one embodiment of a chest compression device according to the present invention;
Fig. 4 is a front view of one embodiment of the chest compression device according to the present invention;
Fig. 5 is a cross-sectional view for explaining the operation of the chest compression device according to the present invention;
Fig. 6 is a cross-sectional view illustrating a coupling relation of one embodiment of the chest compression device according to the present invention; and
Fig. 7 is a cross-sectional view illustrating a coupling relation of another embodiment of the chest compression device according to the present invention.

### Best means for carrying out the invention

Hereinafter, preferred examples of the present invention will be described in detail to help the understanding of the present invention. However, the following examples are merely illustrative of the contents of the present invention, and the scope of the present invention is not limited to the following examples. The examples of the present invention are provided to explain the present invention more completely to those of ordinary skill in the art.

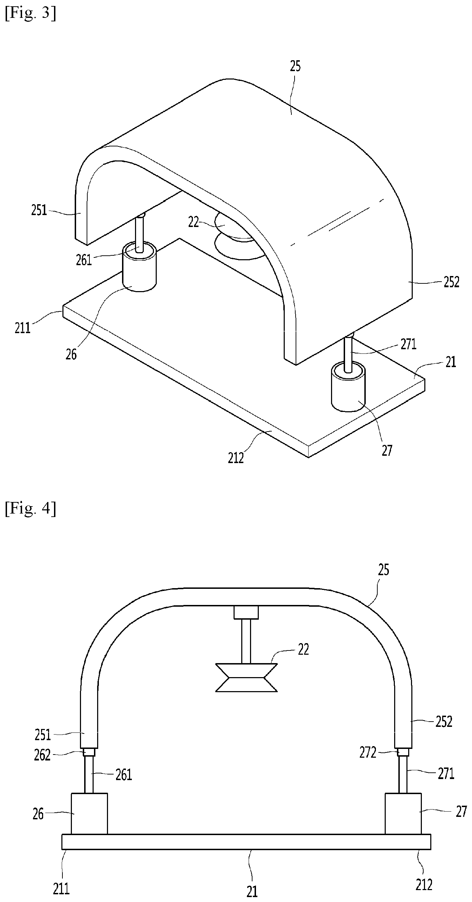

Figs. 3 and 4 are a perspective view and a front view of the chest compression device according to the present invention.

The chest compression device of the present invention includes a back 21 for supporting the back of a patient; a compression part 22 for compressing the chest of the patient; a U-shaped support 25 for supporting the compression part 22; a pair of driving parts 26, 27 for allowing the support 25 and the compression part 22 connected thereto to perform a vertical reciprocating movement; and a control part (not illustrated) for controlling the operation of the pair of driving parts 26, 27.

In the chest compression device, one end of the first driving part 26 among the pair of driving parts is connected to one end 251 of the support 25, and the other end of the first driving part 26 is connected to one end 211 of the back 21. One end of the second driving part 27 among the pair of driving parts is connected to the other end 252 of the support 25, and the other end of the second driving part 27 is connected to the other end 212 of the back 21.

For example, the pair of driving parts 26, 27 is respectively provided with rods 261, 271 capable of performing a vertical linear movement, and is supported by the back 21.

Here, the distal end of the rod 261 of the first driving part 26 among the pair of driving parts is connected to one end 251 of the support 25 (for example, may be connected by a fastening member 262), and the first driving part 26 is connected to and supported by one end 211 of the back 21. Likewise, the distal end of the rod 271 of the second driving part 27 among the pair of driving parts is connected to the other end 252 of the support 25 (for example, may be connected by a fastening member 272), and the second driving part 27 is connected to and supported by the other end 212 of the back 21.

The compression part 22 is fixed in the center of the support 25, and the control part (not illustrated) may control the rate, distance, pressure, etc. of the vertical reciprocating movement of the support 25 through wired or wireless communications with the pair of driving parts 26, 27. Therefore, the control part (not illustrated) may control the rate, compression depth, pressure, etc. of chest compression on the patient performed by the compression part 22 connected to the support 25. Additionally, the control part (not illustrated) may control the vertical reciprocating movements at both ends of the support 25 by the pair of driving parts 26, 27 to be synchronized with each other. Accordingly, the support 25 and the compression part 22 may perform the vertical reciprocating movements in parallel.

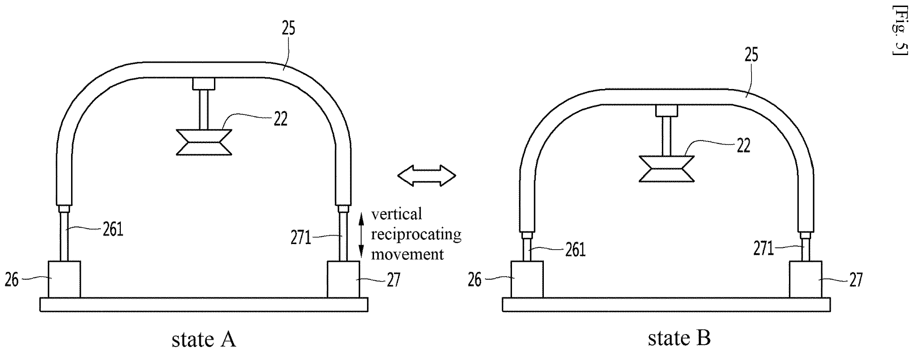

According to the chest compression device of the present invention as illustrated in Fig. 5, the rods 261, 271 of the pair of driving parts 26, 27 are synchronized with each other to perform vertical reciprocating movements, and thus the support 25 and the compression part 22 may perform vertical reciprocating movements in parallel. Accordingly, the chest compression device of the present invention comes and goes between state A and state B so that the compression part 22 can regularly compress the chest of the cardiac arrest patient at a predetermined pressure.

Meanwhile, the support 25 and the compression part 22 of the chest compression device according to the present invention are made of materials through which X-rays may penetrate (for example, plastic materials for the compression part). By using materials through which X-rays may penetrate for the support 25 and the compression part 22, cardiovascular images may be obtained using coronary angiography while CPR is performed with the chest compression device of the present invention. In other words, it is possible to monitor the blood vessels of the heart using angiography simultaneously with chest compression on the cardiac arrest patient using the chest compression device of the present invention.

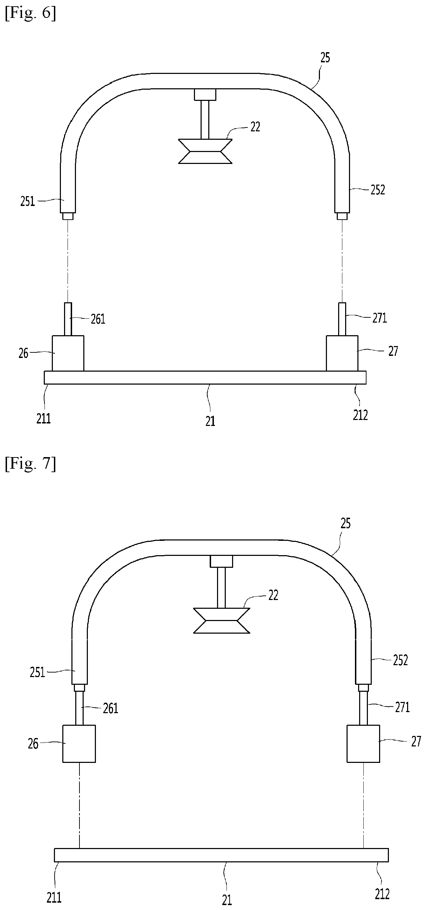

Fig. 6 is a cross-sectional view illustrating a coupling relation of one embodiment of the chest compression device of the present invention. The chest compression device according to the present invention may be used by laying a patient on the back 21, while a pair of driving parts 26, 27 are already fastened into the back 21 for supporting the back of the patient, and connecting both ends 251, 252 of the support 25 provided with the compression part 22 to the pair of driving parts 26, 27.

Fig. 7 is a cross-sectional view illustrating a coupling relation of another embodiment of the chest compression device according to the present invention. Here, the chest compression device may be used by laying a patient on the back 21, while both ends 251, 252 of the support 25 provided with the compression part 22 are connected to a pair of driving parts 26, 27, and connecting and fixing the pair of driving parts 26, 27 to both ends of the back 21 with a fastening member (not illustrated).

### Industrial applicability

By using the chest compression device according to the present invention, it is possible to take images of the blood vessels of a cardiac arrest patient by coronary angiography while performing CPR.

## Claims

1. A chest compression device, comprising:
a back for supporting the back of a patient;
a compression part for compressing the chest of the patient;
a U-shaped support for supporting the compression part;
a pair of driving parts for allowing the support and the compression part connected thereto to perform a vertical reciprocating movement; and
a control part for controlling the operation of the pair of driving parts.

2. The chest compression device of claim 1,
wherein one end of a first driving part among the pair of driving parts is connected to one end of the support, and the other end of the first driving part is connected to one end of the back, and
wherein one end of a second driving part among the pair of the driving parts is connected to the other end of the support, and the other end of the second driving part is connected to the other end of the back.

3. The chest compression device of claim 1, wherein
each of the pair of driving parts is provided with a rod performing a vertical linear movement, and
each of the pair of driving parts is supported by the back.

4. The chest compression device of claim 3,
wherein the distal end of the rod of the first driving part among the pair of driving parts is connected to one end of the support, and the first driving part is connected to and supported by one end of the back, and
wherein the distal end of the rod of the second driving part among the pair of driving parts is connected to the other end of the support, and the second driving part is connected to and supported by the other end of the back.

5. The chest compression device of any one of claims 1 to 4, wherein the support and the compression part are made of materials through which X-rays may penetrate.

6. The chest compression device of claim 5, wherein the support and the compression part are made of plastic.

7. The chest compression device of any one of claims 2 to 4, wherein the control part controls the vertical reciprocating movements of both ends of the support by the pair of driving parts to be synchronized with each other.
